# FASCICULE DE BREVET EUROPEEN

(11) **EP 1 129 640 B1**
(45) Date de publication et mention de la délivrance du brevet: **08.10.2003**
(21) Numéro de dépôt: 01400553.2
(22) Date de dépôt: 02.03.2001
(51) Int. Cl.: A45D 40/00, A45D 40/26, B05C 1/00, B05C 17/00

(54) **Dispositif comprenant un applicateur et/ou un organe d'essorage magnétique**
Vorrichtung mit einem magnetischen Auftragelement und/oder Trocknerelement
Device comprising an applicator and/or a magnetic drying device

(30) Priorité: 03.03.2000 FR 0002757
(43) Date de publication de la demande: 05.09.2001
(60) Demande divisionnaire: 03291812.0
(73) Titulaire: L'OREAL, 75008 Paris (FR)
(72) Inventeur: Gueret, Jean-Louis, 75016 Paris (FR)
(74) Mandataire: Tanty, François

(56) Documents cités:
- EP-A- 1 043 018
- WO-A-92/14435
- DE-A- 3 619 987
- US-A- 4 940 350
- US-A- 5 961 988

## Description

La présente invention concerne l'application d'un produit, notamment cosmétique, sur une région du corps ou du visage ou sur les cheveux.

Il est de plus en plus fréquent d'incorporer dans les produits cosmétiques un ou plusieurs actifs destinés au soin de la surface traitée.

Il peut s'agir, par exemple, d'actifs hydratants ou antirides.

Il existe un besoin notamment pour favoriser la pénétration de ces actifs et renforcer ainsi leur efficacité.

On a proposé dans la demande de brevet DE 4 325 071 d'utiliser des particules magnétiques pour favoriser la microcirculation.

Dans cette demande antérieure, les particules sont dispersées dans une crème, lotion ou gel ou déposées sur un bandage.

Un problème qui se pose lors de l'utilisation de particules magnétiques est leur tendance à s'agglomérer.

On connaît par la publication WO92/14435 un dispositif de massage ayant une tête dans laquelle peut être intégré un aimant.

Cette tête n'est pas prévue pour pouvoir être chargée en produit sur une certaine profondeur et le transférer sur la surface à traiter.

L'invention vise notamment à résoudre ce problème et a pour objet un nouveau dispositif pour l'application d'un produit, notamment cosmétique, comportant un récipient et un applicateur agencé pour prélever du produit dans ce récipient, l'applicateur comprenant une structure servant à l'application et capable de transporter du produit sur la surface à traiter et, éventuellement, un organe d'essorage pour essorer cet applicateur avant l'application du produit, qui soit capable d'exercer au moyen d'un champ magnétique une action bénéfique sur la surface traitée, par exemple sur la microcirculation ou d'autres aspects du métabolisme. Un tel dispositif est connu par US-A-4940350

L'invention y parvient grâce aux caractéristiques énoncées dans la revendication 1.

Au sens de la présente demande, la structure d'application doit se comprendre comme une structure apte à être chargée en produit à appliquer notamment sur une certaine profondeur plus ou moins importante, et à transférer le produit sur une surface à traiter lorsque la structure est déplacée et/ou comprimée sur ladite surface.

A l'application, la structure d'application conserve son intégrité.

Le dispositif selon l'invention présente avantageusement une structure unitaire autonome, c'est à dire qu'il n'est pas relié par un tuyau à un récipient ou par un câble électrique à une source d'alimentation électrique, à la différence du dispositif décrit dans la demande WO92/14435.

Le ou les éléments générant, ou apte à générer un champ magnétique d'orientation prédéterminée sont, selon l'invention, disposés au coeur même de la structure d'application.

La profondeur de pénétration du produit dans la structure servant à l'application dépend de la nature de celle-ci, et peut se limiter à l'épaisseur d'un flocage par exemple.

L'invention permet, lorsque l'applicateur est magnétique, de favoriser la pénétration d'un ou plusieurs actifs contenus dans le produit en améliorant la microcirculation dans le voisinage de l'applicateur.

L'invention permet de plus d'utiliser des particules magnétiques sans craindre qu'elles ne forment des agrégats, puisque ces particules sont immobilisées au sein de la structure servant à l'application du produit.

Le produit présent sur l'applicateur peut acquérir, du fait de son exposition au champ magnétique, des propriétés additionnelles, par exemple se polariser et présenter un pouvoir pénétrant accru.

Dans le cas où seul l'organe d'essorage est magnétique, de telles propriétés additionnelles peuvent être acquises au passage à travers l'organe d'essorage.

Dans une réalisation préférée, la structure précitée comporte un ou plusieurs aimants et/ou des particules magnétiques et/ou magnétisables.

Avantageusement, le dispositif comporte un récipient et l'applicateur est agencé pour prélever du produit dans ce récipient.

Dans une réalisation particulière, l'applicateur est agencé pour que du produit puisse être pompé dans la structure servant à l'application.

Dans une autre réalisation particulière, la structure servant à l'application du produit est agencée de manière à être traversée par du produit provenant du récipient.

Dans une autre réalisation particulière encore, la structure servant à l'application du produit est pré-imprégnée de produit, auquel cas l'applicateur est conditionné par exemple dans une enveloppe étanche et sert d'échantillon.

Avantageusement, la structure servant à l'application du produit et/ou l'organe d'essorage sont au moins partiellement poreux.

Dans une réalisation préférée, la structure précitée et/ou l'organe d'essorage sont entièrement poreux.

La structure servant à l'application du produit et/ou l'organe d'essorage sont avantageusement réalisés au moyen d'au moins un matériau choisi dans la liste suivante : mousse, éponge, fritté, agrégats de fibres naturelles ou synthétiques, tissés, non tissés, ou une combinaison de ces matériaux.

Avantageusement, on utilise des particules magnétiques et/ou magnétisables incorporées dans un tel matériau au cours de son élaboration, ce qui permet de lier intimement ou de faire adhérer les particules magnétiques et/ou magnétisables et les fibres ou cellules dudit matériau.

On s'assure ainsi que les particules magnétiques et/ou magnétisables restent solidaires du matériau constituant la structure servant à l'application du produit et ne risquent pas de quitter l'applicateur ou l'organe d'essorage pour se déposer sur la surface traitée, ce qui pourrait être source d'inconfort.

Dans une réalisation particulière, la structure servant à l'application du produit et/ou l'organe d'essorage comportent une mousse ou une éponge présentant au moins 10 % de cellules ouvertes.

L'épaisseur d'une telle structure peut être d'au moins quelques millimètres, par exemple au moins 5 millimètres.

La structure servant à l'application du produit et/ou l'organe d'essorage peuvent comporter en surface un flocage et/ou des aspérités.

Avantageusement, la structure servant à l'application du produit est fixée sur un support, notamment par sur-injection de matière, soudage ou collage.

Un tel support peut servir d'organe de préhension.

Le support peut comporter un ou plusieurs aimants et/ou des particules magnétiques, afin par exemple de renforcer le champ magnétique exercé sur la surface traitée et/ou de générer des champs magnétiques d'orientations différentes.

De préférence, la structure servant à l'application du produit et/ou l'organe d'essorage comportent des particules dispersées, avantageusement de façon sensiblement homogène.

La dispersion homogène des particules peut résulter du fait que celles-ci sont incorporées dans le matériau servant à réaliser la structure servant à l'application du produit et/ou l'organe d'essorage à l'état non magnétisé, au cours de la fabrication *de* ce matériau.

La structure servant à l'application du produit et/ou l'organe d'essorage peuvent comporter entre 0,2 et 80 % en poids de particules magnétiques.

La structure servant à l'application du produit et/ou l'organe d'essorage peuvent comporter un assemblage de plusieurs types de matériaux poreux, afin de conférer par exemple à l'applicateur une rigidité variable selon certaines zones et permettre de traiter différemment une surface en fonction de la partie de l'applicateur utilisée pour un tel traitement.

La structure servant à l'application du produit et/ou l'organe d'essorage peuvent comporter des particules magnétiques et/ou des aimants agencés de manière à générer des champs magnétiques d'orientations différentes.

En particulier, lorsque l'on utilise plusieurs matériaux poreux assemblés pour constituer l'organe d'essorage ou la structure servant à l'application du produit, on peut incorporer dans chacun de ces matériaux des particules magnétiques et/ou aimants générant un champ d'orientation prédéterminée et combiner les différents matériaux de manière à obtenir le champ résultant recherché.

On peut utiliser comme particules magnétiques des ferrites, notamment ferromagnétiques à champ coercitif élevé.

On peut également utiliser des ferrites paramagnétiques situées dans le voisinage d'un aimant générant un champ magnétique d'aimantation de ces particules.

L'invention a encore pour objet un procédé pour fabriquer un applicateur ou un organe d'essorage destiné à essorer un applicateur, ce procédé étant caractérisé par le fait qu'il comporte les étapes suivantes :
a) incorporer à l'intérieur de l'organe d'essorage ou dans la structure d'application de l'applicateur des particules magnétisables,
b) soumettre lesdites particules à un champ magnétique de manière à ce que ladite structure génère ou soit apte à générer au moins un champ magnétique d'orientation prédéterminée.

Le champ magnétique servant à aimanter les particules magnétisables peut être généré par un aimant faisant partie de l'applicateur, ce qui peut éviter d'avoir à soumettre l'applicateur ou l'organe d'essorage à un banc d'aimantation.

Le fait d'introduire les particules à l'état non aimanté dans la structure poreuse permet d'éviter les inconvénients liés au risque d'agglomération des particules lorsque celles-ci sont magnétiques.

D'autres caractéristiques et avantages de la présente invention ressortiront à la lecture de la description détaillée qui va suivre, d'exemples de réalisation non limitatifs, et à l'examen du dessin annexé, sur lequel :
- la figure 1 est une vue schématique en coupe axiale d'un dispositif conforme à un premier exemple de réalisation,
- la figure 2 est une vue schématique à échelle agrandie de la mousse de l'applicateur de la figure 1,
- la figure 3 est une coupe axiale schématique illustrant une variante de réalisation de l'applicateur,
- les figures 4 à 7 illustrent des variantes de réalisation de dispositifs conformes à l'invention,
- la figure 8 représente isolément et de manière schématique un applicateur,
- la figure 9 est une section selon le trait de coupe IX-IX de la figure 8,
- la figure 10 représente de manière schématique une variante de réalisation de l'applicateur,
- la figure 11 est une vue schématique à échelle agrandie d'une structure poreuse conforme à une variante de mise en oeuvre de l'invention,
- la figure 12 illustre une variante de réalisation d'un dispositif conforme à l'invention.

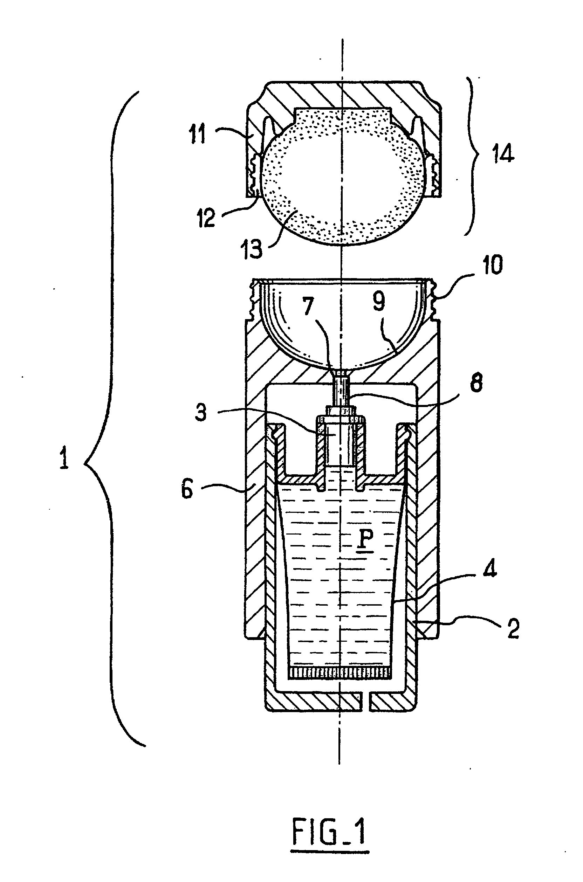

On a représenté sur la figure 1 un dispositif de conditionnement et d'application 1 comportant un corps 2 sur lequel est montée une pompe 3 sans reprise d'air. Cette pompe 3 est agencée pour pomper un produit P, cosmétique ou de soin, contenu dans une poche souple 4 logée dans le corps 2.

Le produit P peut être aqueux ou huileux, contenir des actifs, par exemple hydrosolubles ou liposolubles.

Un capot coulissant 6 est monté sur le corps 2. Ce capot 6 comporte en partie supérieure un col 10 fileté extérieurement et, à l'intérieur de ce dernier, un logement 9 sensiblement hémisphérique, ouvert vers le haut.

Le dispositif de conditionnement et d'application 1 comporte également un applicateur 14 comprenant un capot de fermeture 11, pourvu d'une jupe de montage 12 agencée pour se visser sur le col fileté 10, et une structure d'application 13 constituée par une mousse de caoutchouc naturel (NRB) à cellules ouvertes, fixée à l'intérieur du capot de fermeture 11 et dépassant de celui-ci vers le bas afin de pouvoir être appliquée sur la peau.

Le capot de fermeture 11 sert de support et d'organe de préhension.

Le capot 6 est traversé, dans le fond du logement 9, par un orifice comportant un épaulement 7 contre lequel vient en appui la tige 8 de la pompe 3.

Le col fileté 10 et la jupe de montage 12 sont agencés pour coopérer de manière étanche lorsque le capot de fermeture 11 est en place, de sorte que l'applicateur 14 est alors empêché de sécher.

La structure d'application 13 comporte dans sa masse des particules magnétiques.

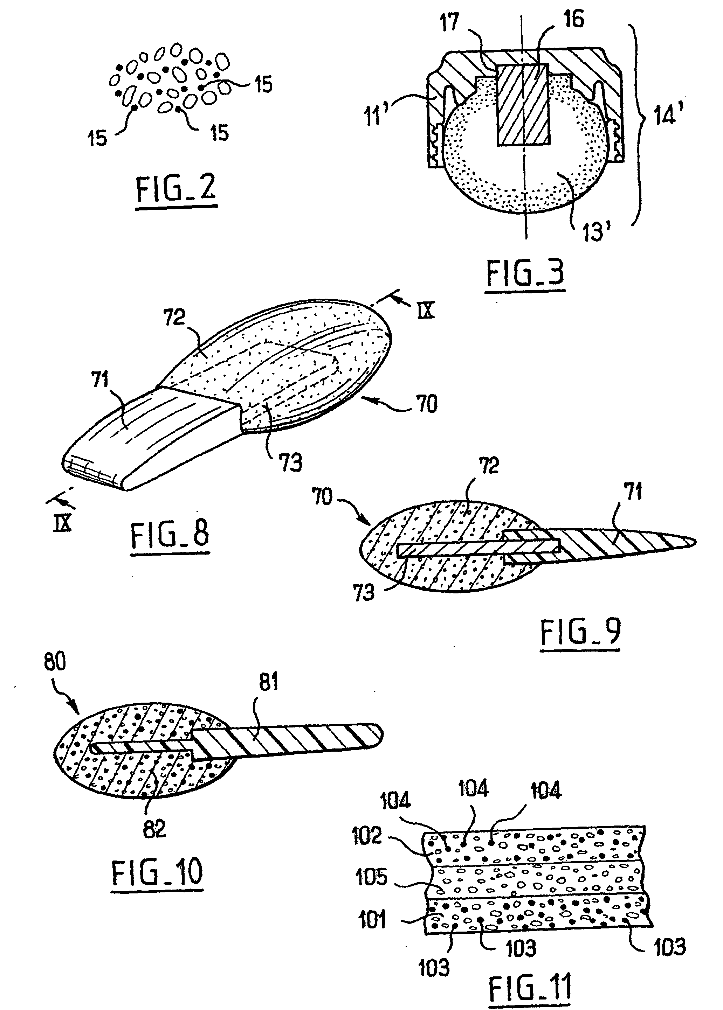

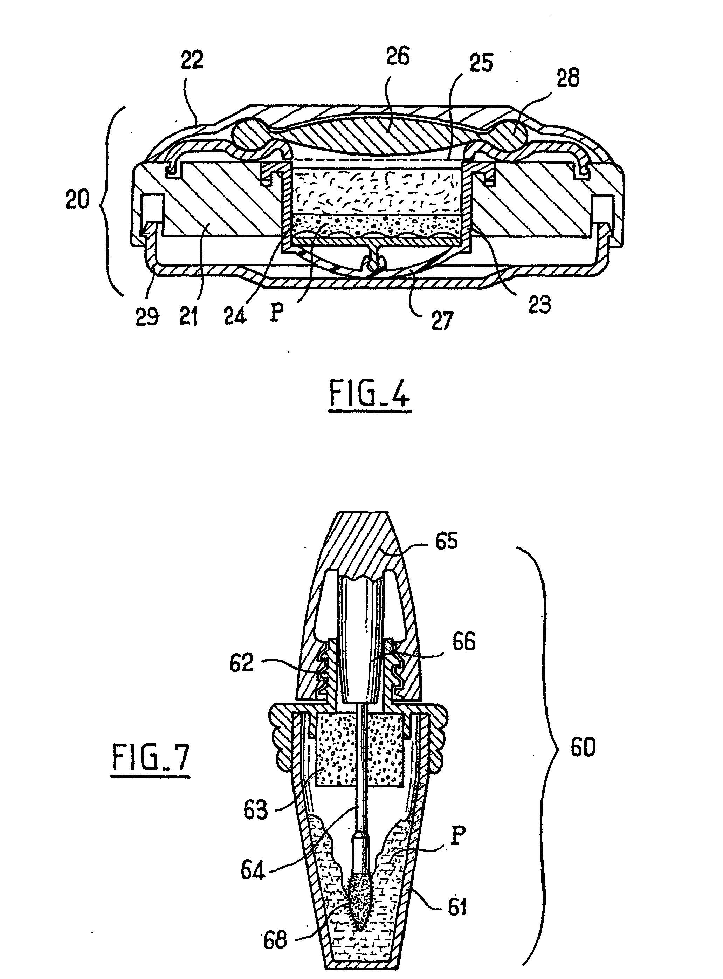

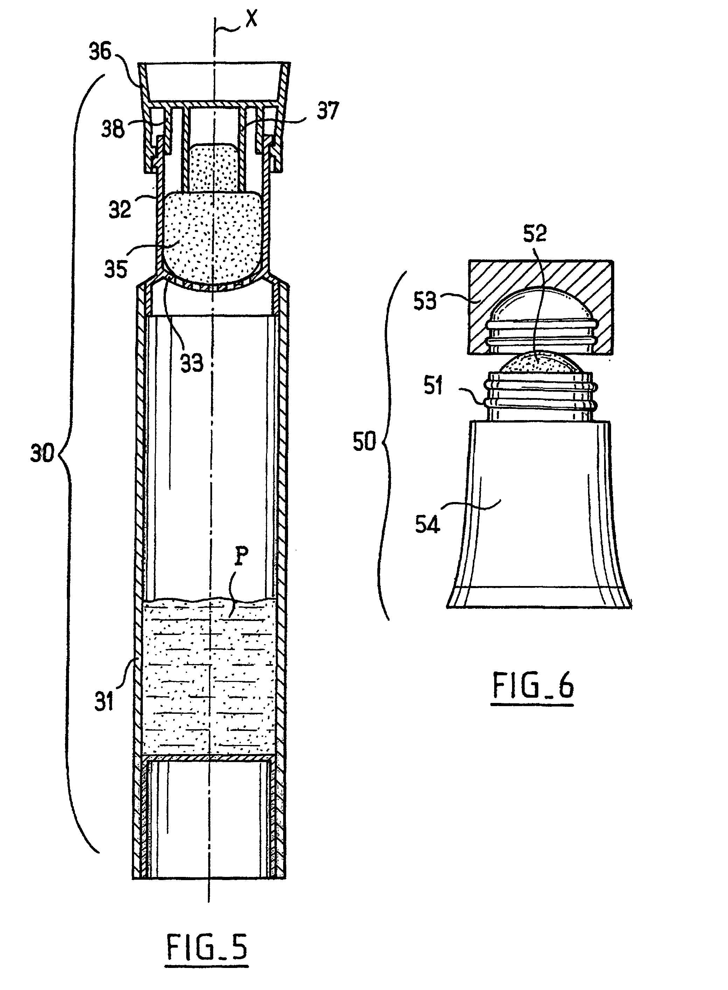

Plus précisément, la mousse constituant la structure d'application 13 comporte, comme illustré sur la figure 2, des particules 15 dispersées en son sein, de manière sensiblement homogène.

De telles particules 15 sont constituées par exemple par des particules ferromagnétiques telles que des ferrites, notamment des ferrites à base de zinc et de manganèse ou autres, capables de conserver une aimantation rémanente notable.

Ces particules 15 sont enrobées dans l'exemple décrit d'un revêtement inerte de polyuréthane.

En variante, on peut les enrober d'élastomère, d'époxy, de polyester, de polyamide, de résine urée formol ou de cyanoacrylate, par exemple, en vue de les protéger de l'oxydation et leur conférer une couleur différente, notamment la même couleur que la structure d'application 13, afin de les rendre indécelables.

Les particules 15 sont introduites à l'état non magnétisé dans la mousse lors de sa fabrication, puis magnétisées une fois que la réaction chimique produisant la mousse est terminée.

Pour réaliser la structure d'application 13, on peut partir d'un pain de mousse comportant des particules magnétisables.

Ce pain de mousse peut être magnétisé en entier après sa fabrication, puis découpé pour former la structure d'application 13.

En variante, le pain de mousse peut être découpé pour former la structure d'application 13 alors que les particules magnétisables ne sont pas encore magnétisées.

L'applicateur 14 est alors magnétisé à part, après sa fabrication.

Les particules 15 présentes dans la mousse peuvent être magnétisées en les soumettant à un champ magnétique généré par un banc de magnétisation tel que ceux figurant au catalogue de la société TE2M (Technique et Matériel Magnétique) sous les références CE500, PM 1000 ou PM 2500, lesquels sont des dispositifs de puissance moyenne à grande, permettant d'aimanter à une cadence élevée des aimants de nuances et de formes diverses.

Après aimantation, les particules 15 se comportent individuellement chacune comme un aimant élémentaire, générant un champ magnétique d'orientation prédéterminée, en fonction des conditions de magnétisation.

En variante, comme illustré sur la figure 3, on utilise un applicateur 14' dans lequel la structure d'application 13 de l'applicateur 14 est remplacée par une structure d'application 13' contenant des particules magnétiques et un aimant permanent 16 fixé sur le capot de fermeture 11', lequel diffère du capot 11 précédemment décrit par la présence d'un renfoncement 17 servant à la fixation de l'aimant 16.

La présence de ce dernier permet d'augmenter l'intensité du champ magnétique exercé par l'applicateur 14', lorsque ce dernier comporte des particules magnétiques 15 comme dans l'exemple précédent.

L'aimant 16 permet également de générer un champ magnétique d'orientation différente de celle du champ magnétique produit par les particules magnétiques contenues dans l'applicateur 13'.

Dans une autre variante de réalisation, la structure d'application 13' est remplacée par une structure d'application comportant des particules magnétisables destinées à être aimantées par l'aimant 16, afin par exemple d'éviter d'avoir à soumettre ces particules à un champ magnétique d'aimantation généré par un banc d'aimantation.

On peut également utiliser, grâce à la présence de l'aimant 16, des particules paramagnétiques, et/ou une combinaison de particules paramagnétiques et/ou ferromagnétiques.

L'invention n'est pas limitée à l'utilisation de ferrites, on peut utiliser comme particules paramagnétiques celles commercialisées par la société CORTEX BIOCHEM sous la dénomination MAGACELL ou par la société norvégienne DYNAL sous la dénomination DYNABEAD.

Dans les exemples de réalisation des figures 1 à 3, la structure d'application 13 ou 13' se charge en produit sous l'effet de la pression du produit délivré par la pompe 3 et, éventuellement par un effet de pompage, lors du retrait de l'applicateur, dû à la détente de la mousse lors du dévissage du capot de fermeture 11 ou 11'.

L'invention n'est pas limitée à ce type d'applicateur.

On a représenté sur la figure 4 un dispositif de conditionnement et d'application 20 comportant un boîtier ayant un corps 21, un couvercle articulé 22 et un fond coulissant 29.

Le corps 21 comporte un logement recevant une coupelle 23 contenant une réserve de produit P.

La coupelle 23 est munie en partie supérieure d'un tamis 25 et comporte en partie inférieure une paroi déformable 27.

Un piston 24 solidaire de la paroi déformable 27 peut coulisser dans la coupelle 23.

En appuyant sur le fond coulissant 29, l'utilisateur repousse le piston 24 vers le haut et chasse du produit à travers le tamis 25.

Un applicateur 26 constitué par une éponge se loge à l'intérieur du couvercle 22 au-dessus du tamis 25.

Le dispositif de conditionnement et d'application 20 est de préférence agencé, comme dans l'exemple décrit, pour assurer la conservation étanche de l'applicateur 26.

A cet effet, l'applicateur 26 est solidaire à sa périphérie d'un bourrelet annulaire 28 qui sert de joint d'étanchéité en étant pincé, lorsque le boîtier est fermé, entre le couvercle 22 et un cache monté sur le corps 21.

L'applicateur 26 est ainsi empêché de sécher lorsque le couvercle 22 est fermé.

La région centrale de l'applicateur 26 constitue une structure d'application capable de se charger en produit et incorpore une dispersion de particules magnétiques aptes à générer un champ magnétique d'orientation donnée.

Dans une variante, l'applicateur 26 est remplacé par un agrégat de fibres naturelles ou synthétiques.

On a représenté sur la figure 5 un dispositif de distribution et de conditionnement 30 qui comporte un récipient 31 pourvu d'un col fileté extérieurement 32.

Le col 32 comporte intérieurement, dans son fond, une paroi perforée 33.

Le dispositif de distribution et de conditionnement 30 comporte un applicateur comprenant une structure d'application 35 portée par un organe de préhension 36 qui constitue également le capuchon de fermeture du col 32.

La structure d'application 35 est partiellement engagée et fixée dans une jupe de montage 37 du capuchon 36.

Ce dernier comporte une jupe d'étanchéité 38 conformée pour s'ajuster de manière étanche dans le col 32 lorsque le capuchon 36 est vissé sur celui-ci.

La structure d'application 35 est constituée par une mousse de polyuréthane à cellules ouvertes et incorpore une dispersion de particules magnétiques.

La structure d'application 35 peut se charger de produit par capillarité.

On a représenté sur la figure 6 un dispositif de distribution et de conditionnement 50 comprenant un tube souple 54 pourvu d'un col fileté 51, ce col étant muni intérieurement d'un applicateur 52 comportant une structure d'application constituée par un bloc de mousse de polyéther à cellules ouvertes.

L'applicateur 52 incorpore des particules magnétiques.

En l'absence d'utilisation, le tube 54 est fermé de manière étanche par un capuchon de fermeture 53.

Pour distribuer du produit, l'utilisateur presse le tube 54.

La distribution de produit s'effectue à travers l'applicateur 52, ce dernier étant perméable au produit contenu dans le tube.

Dans une variante, l'applicateur 52 est remplacé par un fritté de particules en matière plastique, notamment du polyéthylène, en céramique, en métal ou en verre, un tel fritté comportant des pores communiquant entre eux dans toutes les directions.

On a représenté sur la figure 7 un dispositif de conditionnement et d'application 60 comportant un récipient 61 pourvu d'un col 62 fileté extérieurement.

Un organe d'essorage 63 constitué par un bloc de mousse de polyuréthane à cellules ouvertes, fendu axialement, est fixé à l'intérieur du récipient 61 sous le col 62.

Le récipient 61 est fermé par un capuchon de fermeture 65 vissé sur le col 62.

Ce capuchon de fermeture 65 comporte une partie centrale 66 apte à s'ajuster de manière étanche dans le col 62.

La partie centrale 66 se prolonge vers le bas par une tige 64 pourvue en extrémité d'un applicateur floqué 68.

L'organe d'essorage 63 sert à essorer la tige 64 et l'applicateur 68 lorsque ce dernier est extrait du récipient 61.

L'organe d'essorage 63 incorpore des particules magnétiques, lesquelles soumettent à un champ magnétique l'applicateur 68 lorsque celui-ci est extrait du récipient 61.

Cette exposition à un champ magnétique est susceptible d'entraîner des modifications du produit passant au travers de l'organe d'essorage, modifications favorisant par exemple la pénétration du ou des actifs au moment de l'application.

On a représenté sur les figures 8 et 9 un applicateur 70 qui comporte un support 71 en matière plastique rigide ou semi-rigide et une structure d'application 72, par exemple surmoulée sur le support 71, la structure d'application 72 pouvant comporter en outre un flocage en surface.

Le support 71 est agencé pour recevoir un noyau magnétique 73, constitué par exemple par un aimant.

Dans une variante non représentée, le noyau magnétique 73 est remplacé par un prolongement du support 71 dans lequel sont incorporées des particules magnétiques, par exemple des ferrites.

Dans l'exemple des figures 8 et 9, le support 71 sert d'organe de préhension.

Dans une variante non illustrée, le support 71 est remplacé par une tige telle que la tige 64 du dispositif de conditionnement et d'application 60 de la figure 6.

On a représenté sur la figure 10 un applicateur 80 comportant un support 81 et un élément d'application 82 constitué par un matériau poreux, par exemple une mousse, incorporant des particules magnétiques, par exemple des ferrites.

L'invention n'est pas limitée à une structure particulière d'applicateur et l'on peut notamment utiliser comme structure capable de retenir du produit pour l'application une structure composite comportant par exemple, comme illustré sur la figure 11, plusieurs couches ayant des propriétés magnétiques différentes, par exemple deux couches poreuses inférieure 101 et supérieure 102 contenant respectivement des particules magnétiques 103 et 104, séparées par une couche poreuse 105 non magnétique.

La structure multicouche de la figure 11 permet d'exposer la surface à traiter à des champs magnétiques d'orientations différentes, si les champs magnétiques générés par les particules 103 et 104 ont des orientations différentes, et de réaliser un applicateur ayant une rigidité variable.

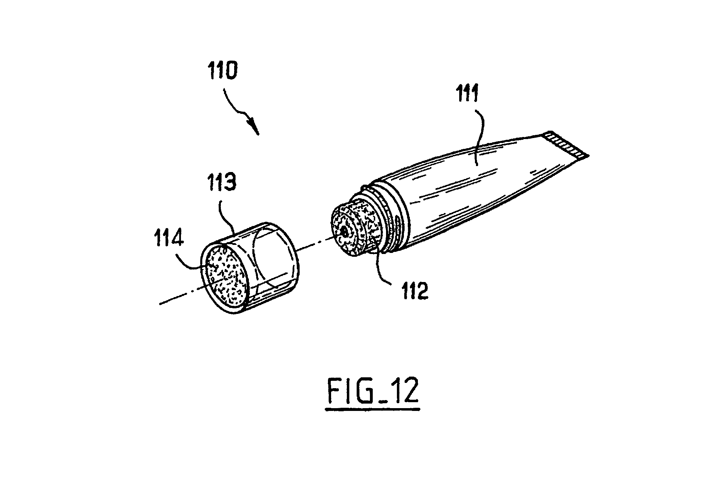

On a représenté sur la figure 12 un dispositif de conditionnement et d'application 110 qui comporte un tube 111 contenant un produit tel qu'un lait, muni d'un applicateur comprenant une structure d'application 112 constituée par un bloc de mousse incorporant des particules magnétiques.

Le dispositif 110 comporte un capot de fermeture 113 logeant une réserve 114 d'un produit capable de se déliter progressivement au contact du produit contenu dans le tube 111.

Le produit traverse la structure d'application 112 pour être délivré et se trouve alors soumis au champ magnétique généré par les particules magnétiques présentes au sein de celle-ci.

L'invention, grâce à l'utilisation de particules magnétiques , permet d'exercer une action bénéfique, notamment sur l'oxygénation de la peau et sur la microcirculation au moment de l'application d'un produit sur la peau, en soumettant celle-ci à l'action d'un champ magnétique.

L'efficacité des actifs contenus dans le produit appliqué se trouve renforcée.

Le ou les champs magnétiques générés peuvent avoir diverses orientations, et être notamment d'axe polaire N - S perpendiculaire ou parallèle à la surface traitée.

Lorsque la structure d'application comportant des particules magnétiques est capable de se déformer au moment de l'application, l'orientation du champ magnétique peut être modifiée sous l'effet des déformations de la structure d'application.

## Revendications

1. Dispositif pour l'application d'un produit, notamment cosmétique, comportant un récipient et un applicateur agencé pour prélever du produit dans ce récipient et comprenant une structure servant à l'application et capable de transporter du produit sur une surface à traiter et, éventuellement, un organe d'essorage pour essorer cet applicateur avant l'application du produit, **caractérisé par le fait qu'**au moins une partie de ladite structure (14 ; 14' ; 35 ; 52 ; 72 ; 82 ; 26 ; 112 ; 120) et/ou de l'organe d'essorage (63) comporte des particules magnétiques et/ou magnétisables dispersées générant ou aptes à générer un champ magnétique d'orientation prédéterminée.

2. Dispositif selon la revendication précédente, **caractérisé par le fait que** l'applicateur est agencé pour que du produit puisse être pompé dans ladite structure.

3. Dispositif selon l'une des revendications 1 et 2, **caractérisé par le fait que** ladite structure est agencée de manière à être traversée par du produit provenant de ce récipient.

4. Dispositif selon l'une quelconque des revendications précédentes, **caractérisé par le fait que** ladite structure est pré-imprégnée de produit.

5. Dispositif selon la revendication précédente, **caractérisé par le fait que** l'applicateur est conditionné dans une enveloppe étanche.

6. Dispositif selon l'une quelconque des revendications précédentes, **caractérisé par le fait que** ladite structure et/ou l'organe d'essorage sont au moins partiellement poreux.

7. Dispositif selon la revendication précédente, **caractérisé par le fait que** ladite structure et/ou l'organe d'essorage sont entièrement poreux.

8. Dispositif selon l'une quelconque des revendications précédentes, **caractérisé par le fait que** ladite structure et/ou l'organe d'essorage sont réalisés au moyen d'au moins un matériau choisi dans la liste suivante : mousse, éponge, fritté, agrégats de fibres naturelles ou synthétiques, tissés, non tissés, ou une combinaison de ces matériaux.

9. Dispositif selon la revendication précédente, ladite structure et/ou l'organe d'essorage comportant une mousse ou une éponge, **caractérisé par le fait que** cette mousse ou cette éponge présente au moins 10 % de cellules ouvertes.

10. Dispositif selon l'une quelconque des revendications précédentes, **caractérisé par** le fait ladite structure et/ou l'organe d'essorage comportent en surface un flocage et/ou des aspérités.

11. Dispositif selon l'une quelconque des revendications précédentes, **caractérisé par le fait que** ladite structure est fixée sur un support, notamment par sur-injection de matière, soudage ou collage.

12. Dispositif selon la revendication précédente, **caractérisé par le fait que** ledit support sert d'organe de préhension.

13. Dispositif selon l'une des deux revendications précédentes, **caractérisé par le fait que** ledit support comporte un ou plusieurs aimants et/ou des particules magnétiques.

14. Dispositif selon la revendication précédente, **caractérisé par le fait que** lesdites particules magnétiques sont dispersées de façon sensiblement homogène dans ladite structure ou dans l'organe d'essorage.

15. Dispositif selon l'une quelconque des revendications précédentes, **caractérisé par le fait que** ladite structure et/ou l'organe d'essorage comportent entre 0,2 et 80 % en poids de particules magnétiques.

16. Dispositif selon l'une quelconque des revendications précédentes, **caractérisé par le fait que** ladite structure et/ou l'organe d'essorage comportent un assemblage de plusieurs types de matériaux poreux (101, 102).

17. Dispositif selon l'une quelconque des revendications précédentes, **caractérisé par le fait que** ladite structure et/ou l'organe d'essorage comportent des particules magnétiques et/ou des aimants agencés de manière à générer des champs magnétiques d'orientations différentes.

18. Dispositif selon l'une quelconque des revendications précédentes, **caractérisé par le fait que** les particules magnétiques et/ou magnétisables sont enrobées d'un matériau apte à les protéger de l'oxydation et/ou à changer leur couleur.

19. Dispositif selon l'une quelconque des revendications précédentes, **caractérisé par le fait qu'**il présente une structure unitaire autonome.

20. Procédé pour fabriquer un dispositif comportant un récipient et un applicateur ou un organe d'essorage destiné à essorer un applicateur, **caractérisé par le fait qu'**il comporte les étapes suivantes :
a) incorporer à l'intérieur de l'organe d'essorage ou dans la structure d'application de l'applicateur des particules magnétisables,
b) soumettre lesdites particules à un champ magnétique de manière à ce que ladite structure génère ou soit apte à générer au moins un champ magnétique d'orientation prédéterminée.

21. Procédé selon la revendication précédente, **caractérisé par le fait que** le champ magnétique servant à aimanter les particules magnétisables est généré par un aimant (16) faisant partie de l'applicateur.

## Claims

1. A device for applying a substance, in particular a cosmetic, comprising a receptacle and an applicator arranged to take substance from the receptacle and having a structure for application purposes and capable of transporting the substance onto a surface to be treated, and optionally a wiper member for wiping said applicator prior to applying the substance, **characterized by** the fact that inside at least a portion of said structure (14; 14'; 35; 52; 72; 82; 26; 112; 120) and/or the wiper member (63) there are dispersed magnetic particles and/or magnetizable particles generating or suitable for generating a magnetic field of predetermined orientation.

2. A device according to the preceding claim, **characterized by** the fact that the applicator is arranged so that the substance can be pumped into said structure.

3. A device according to claim 1 or 2, **characterized by** the fact that said structure is arranged in such a manner as to have the substance coming from said receptacle pass therethrough.

4. A device according to any preceding claim, **characterized by** the fact that said structure is preimpregnated in substance.

5. A device according to the preceding claim, **characterized by** the fact that the applicator is packaged in a sealed envelope.

6. A device according to any preceding claim, **characterized by** the fact that said structure and/or the wiper member is/are porous, at least in part.

7. A device according to the preceding claim, **characterized by** the fact that said structure and/or wiper member is/are entirely porous.

8. A device according to any preceding claim, **characterized by** the fact that said structure and/or wiper member is/are made by means of a material selected from the following list: foam, sponge, sintered block, aggregate of natural or synthetic fibers, woven or nonwoven fibers, or a combination of such materials.

9. A device according to the preceding claim, in which said structure and/or wiper member comprise a foam or a sponge and **characterized by** the fact that said foam or said sponge has at least 10% open cells.

10. A device according to any preceding claim, **characterized by** the fact that said structure and/or the wiper member has/have flocking and/or roughness on the surface.

11. A device according to any preceding claim, **characterized by** the fact that said structure is fixed on a support, in particular by overmolding material, by heat-sealing, or by adhesive.

12. A device according to the preceding claim, **characterized by** the fact that said support serves as a handle.

13. A device according to either one of the two preceding claims, **characterized by** the fact that said support includes one or more magnets and/or magnetic particles.

14. A device according to the preceding claim, **characterized by** the fact that said magnetic particles are dispersed in substantially uniform manner throughout said structure or said wiper member.

15. A device according to any preceding claim, **characterized by** the fact that said structure and/or wiper member include(s) 0.2% to 80% by weight magnetic particles.

16. A device according to any preceding claim, **characterized by** the fact that said structure and/or wiper member comprise(s) a plurality of types of porous material (101, 102) assembled together.

17. A device according to any preceding claim, **characterized by** the fact that said structure and/or wiper member include(s) magnetic particles and/or magnets arranged in such a manner as to generate magnetic fields of different orientations.

18. A device according to any preceding claim, **characterized by** the fact that the magnetic and/or magnetizable particles are coated in a material suitable for protecting them against oxidation and/or for changing their color.

19. A device according to any preceding claim, **characterized by** the fact that it presents a self-contained one-piece structure.

20. A method of manufacturing a device comprising a receptacle and an applicator or a wiper member for wiping an applicator, the method being **characterized by** the fact that it comprises the following steps:
a) incorporating magnetizable particles within the wiper member or the applicator structure of the applicator; and
b) subjecting said particles to a magnetic field in such a manner as to cause said structure to generate or be suitable for generating at least one magnetic field of predetermined orientation.

21. A method according to the preceding claim, **characterized by** the fact that the magnetic field for magnetizing the magnetizable particles is generated by a permanent magnet (16) forming part of the applicator.

## Patentansprüche

1. Vorrichtung zum Auftragen eines insbesondere kosmetischen Produkts, umfassend einen Behälter und ein Auftragorgan, das ausgebildet ist, um Produkt in diesem Behälter zu entnehmen, und eine Struktur aufweist, die zum Auftragen dient und in der Lage ist, Produkt auf eine zu behandelnde Oberfläche zu befördern, und ggf. ein Abstreiforgan, um dieses Auftragorgan vor dem Auftrag des Produkts abzustreifen, **dadurch gekennzeichnet, daß** mindestens ein Teil dieser Struktur (14; 14'; 35; 52; 72; 82; 26; 112; 120) und/oder des Abstreiforgans (63) magnetische und/oder magnetisierbare verteilte Teilchen umfaßt, die ein Magnetfeld von vorbestimmter Richtung erzeugen oder erzeugen können.

2. Vorrichtung nach dem vorhergehenden Anspruch, **dadurch gekennzeichnet, daß** das Auftragorgan ausgebildet ist, damit das Produkt in die Struktur gepumpt werden kann.

3. Vorrichtung nach einem der Ansprüche 1 und 2, **dadurch gekennzeichnet, daß** diese Struktur so ausgebildet ist, daß sie von Produkt durchquert wird, das von diesem Behälter kommt.

4. Vorrichtung nach einem der vorhergehenden Ansprüche, **dadurch gekennzeichnet, daß** diese Struktur mit Produkt vorgetränkt ist.

5. Vorrichtung nach dem vorhergehenden Anspruch, **dadurch gekennzeichnet, daß** das Auftragorgan in einer dichten Hülle verpackt ist.

6. Vorrichtung nach einem der vorhergehenden Ansprüche, **dadurch gekennzeichnet, daß** diese Struktur und/oder das Abstreiforgan mindestens teilweise porös sind.

7. Vorrichtung nach dem vorhergehenden Anspruch, **dadurch gekennzeichnet, daß** diese Struktur und/oder das Abstreiforgan ganz porös sind.

8. Vorrichtung nach einem der vorhergehenden Ansprüche, **dadurch gekennzeichnet, daß** diese Struktur und/oder das Abstreiforgan mit Hilfe mindestens eines Werkstoffs hergestellt sind, der aus der folgenden Liste ausgewählt ist: Schaum, Schwamm, Sintermaterial, Aggregate von natürlichen oder synthetischen Fasern, Gewebe, Vliese oder eine Kombination dieser Werkstoffe.

9. Vorrichtung nach dem vorhergehenden Anspruch, wobei diese Struktur und/oder das Abstreiforgan einen Schaum oder einen Schwamm umfassen, **dadurch gekennzeichnet, daß** dieser Schaum oder dieser Schwamm mindestens 10% offene Zellen aufweist.

10. Vorrichtung nach einem der vorhergehenden Ansprüche, **dadurch gekennzeichnet, daß** diese Struktur und/oder das Abstreiforgan auf der Oberfläche eine Beflockung und/oder Unebenheiten aufweist.

11. Vorrichtung nach einem der vorhergehenden Ansprüche, **dadurch gekennzeichnet, daß** diese Struktur auf einem Träger insbesondere durch Aufspritzung von Werkstoff, Verschweißung oder Verklebung befestigt ist.

12. Vorrichtung nach dem vorhergehenden Anspruch, **dadurch gekennzeichnet, daß** der Träger als Greiforgan dient.

13. Vorrichtung nach einem der beiden vorhergehenden Ansprüche, **dadurch gekennzeichnet, daß** der Träger einen oder mehrere Magnete und/oder magnetische Teilchen aufweist.

14. Vorrichtung nach dem vorhergehenden Anspruch, **dadurch gekennzeichnet, daß** die magnetischen Teilchen im wesentlichen homogen in dieser Struktur oder in dem Abstreiforgan verteilt sind.

15. Vorrichtung nach einem der vorhergehenden Ansprüche, **dadurch gekennzeichnet, daß** diese Struktur und/oder das Abstreiforgan zwischen 0,2 und 80 Gew.-% magnetische Teilchen umfassen.

16. Vorrichtung nach einem der vorhergehenden Ansprüche, **dadurch gekennzeichnet, daß** diese Struktur und/oder das Abstreiforgan eine Verbindung von mehreren Typen von porösen Werkstoffen (101, 102) umfassen.

17. Vorrichtung nach einem der vorhergehenden Ansprüche, **dadurch gekennzeichnet, daß** diese Struktur und/oder das Abstreiforgan magnetische Teilchen und/oder Magnete umfassen, die so ausgebildet sind, daß sie Magnetfelder mit verschiedenen Richtungen erzeugen.

18. Vorrichtung nach einem der vorhergehenden Ansprüche, **dadurch gekennzeichnet, daß** die magnetischen und/oder magnetisierbaren Teilchen mit einem Werkstoff umhüllt sind, der geeignet ist, sie gegen Oxidation zu schützen und/oder ihre Farbe zu ändern.

19. Vorrichtung nach einem der vorhergehenden Ansprüche, **dadurch gekennzeichnet, daß** sie eine autonome einheitliche Struktur besitzt.

20. Verfahren zur Herstellung einer Vorrichtung, die einen Behälter und ein Auftragorgan oder ein Abstreiforgan umfaßt, das dazu bestimmt ist, ein Auftragorgan abzustreifen, **dadurch gekennzeichnet, daß** es die folgenden Schritte umfaßt:
a) in das Innere des Abstreiforgans oder in die Auftragstruktur des Auftragorgans magnetisierbare Teilchen einarbeiten,
b) diese Teilchen einem Magnetfeld aussetzen, so daß diese Struktur mindestens ein Magnetfeld mit vorbestimmter Richtung erzeugt oder erzeugen kann.

21. Verfahren nach dem vorhergehenden Anspruch, **dadurch gekennzeichnet, daß** das Magnetfeld, das zur Magnetisierung der magnetisierbaren Teilchen dient, durch einen Magnet (16) erzeugt wird, der Teil des Auftragorgans bildet.
